Europäisches Patentamt

European Patent Office

Office européen des brevets

(11) Veröffentlichungsnummer: **0 165 566 B1**

(12) **EUROPÄISCHE PATENTSCHRIFT**

(45) Veröffentlichungstag der Patentschrift: **03.06.92**

(21) Anmeldenummer: **85107335.3**

(22) Anmeldetag: **13.06.85**

Die Akte enthält technische Angaben, die nach dem Eingang der Anmeldung eingereicht wurden und die nicht in dieser Patentschrift enthalten sind.

(51) Int. Cl.⁵: **A61N 1/365**

(54) **Regelschaltung zur Anpassung der Stimulationsfrequenz eines Herzschrittmachers an die Belastung eines Patienten.**

(30) Priorität: **20.06.84 DE 3422913**

(43) Veröffentlichungstag der Anmeldung:
**27.12.85 Patentblatt 85/52**

(45) Bekanntmachung des Hinweises auf die
Patenterteilung:
**03.06.92 Patentblatt 92/23**

(84) Benannte Vertragsstaaten:
**DE FR GB IT NL SE**

(56) Entgegenhaltungen:
**EP-A- 0 017 848**
**DE-A- 3 243 094**

**FRöHR, ORTTENBURGER "Einführung in die elektronische Regelungstechnik", 4. Auflage, 1976; VERL. SIEMENS AKTIENGESELL-SCHAFT, Berlin und München, Seiten 11-20**

**K. SEIDL "Regeltechnik", 1950; VERL. FRANZ DEUTICKE, Wien, Seiten 2-7**

**TIETZE, SCHENK "Halbleiter-Schaltungstechnik", 5. Auflage, 1980; SPRINGER VERLAG, Berlin, Heidelberg,**

**New York, Seiten 688-695**

(73) Patentinhaber: **Siemens Elema AB
Röntgenvägen 2
S-171 95 Solna 1(SE)**

(84) Benannte Vertragsstaaten:
**SE**

(73) Patentinhaber: **SIEMENS AKTIENGESELL-SCHAFT
Wittelsbacherplatz 2
W-8000 München 2(DE)**

(84) Benannte Vertragsstaaten:
**DE FR GB IT NL**

(72) Erfinder: **Wirtzfeld, Alexander, Prof. Dr.
Hauptstrasse 26
W-8195 Thanning(DE)**
Erfinder: **Heinze, Roland, Dipl.-Ing.
Albertus-Magnus-Weg 5
W-8012 Ottobrunn(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Regelschaltung zur Anpassung der Stimulationsfrequenz eines Herzschrittmachers an die Belastung eines Patienten gemäss dem Oberbegriff des Anspruches 1.

Eine derartige Regelschaltung ist beispielsweise aus der US-A-4 399 820 bekannt. Dabei werden der zentralvenösen Blutsauerstoffsättigung im Herzen entsprechende Messgrössen $S_{02}$ nach dem Prinzip der Reflexionsoximetrie ermittelt und daraus eine Regelgrösse abgeleitet, indem Änderungen dieser Messgrösse pro Zeiteinheit normiert werden auf die maximale Änderung der Messgrösse über lange Zeitabstände, wie Stunden oder Tage.

Mit dieser Regelschaltung ist bereits eine langfristige, ungestörte Messwerterfassung und eine gute hämodynamische Situation im Blutkreislauf erzielbar. Die Nachlaufregelung wird dabei so vorgenommen, dass, wenn die Regelgrösse einen vorgebbaren wert überschreitet, jeweils die Frequenz um einen konstanten Betrag $\Delta f$ verändert wird. Weiterhin ist aus dieser Veröffentlichung bekannt, an die Nachlaufregelung eine optimierende Regelung anzuschliessen, die immer dann einsetzt, wenn während eines vorgebbares Zeitintervalles durch die Nachlaufregelung keine Frequenzänderung vorgenommen wurde.

Die bekannte Regelschaltung nutzt zeitliche Änderungen der normierten Messgrösse als Regelsignal. Das hat zur Folge, dass die Frequenzänderungen zeitabhängig sind und die Stimulationsfrequenz sich abhängig von der Dauer der Änderung der Blutsauerstoffsättigung ändert. Gleiche Gesamtänderungen der Blutsauerstoffsättigung können daher, je nachdem, wie schnell sie erfolgen, zu unterschiedlichen Änderungen der Herzfrequenz führen. Das kann in ungünstigen Fällen dazu führen, dass die Nachlaufregelung physiologisch nicht optimal angepasste Werte erreicht. Diese werden dann jeweils erst durch die anschliessende optimierende Regelung ausgeglichen. Unter Umständen kann das jedoch zu Instabilen Zuständen führen, wobei die Herzfrequenz als Folge periodisch schwankender Belastungszustände zwischen den vorgegebenen Grenzwerten oszilliert. Weiterhin hat sich gezeigt, dass die zeitliche Trennung der beiden Regelvorgänge, d.h. der Nachlaufregelung und der optimierenden Regelung, mit dazwischenliegendem Zeitintervall zur Folge haben kann, dass bei kurzfristigen Änderungen der Blutsauerstoffsättigung, die einem eingeschwungenen Zustand überlagert sind und eine Frequenzänderung über die Nachlaufregelung hervorrufen, die optimierende Regelung verhindern.

Neben diesem Regelverfahren, bei dem zeitliche Änderungen der Messgrösse als Regelgrösse genutzt werden, ist aus der US-A-4 202 339 ein Verfahren bekannt, bei dem die Frequenzanpassung des Herzschrittmachers über eine Kennlinie, d.h. mit fester Zuordnung der Schrittmacherfrequenz $f_p$ zum jeweils ermittelten Messwert der Blutstoffsättigung ist

$$f_p = k \cdot S_{02}, \quad f_{min} < f_p < f_{max}$$

Dabei sind die Werte für $k$, $f_{min}$ und $f_{max}$ vor der Implantation fest eingestellt. Dieses Regelverfahren zeigt eine Reihe von Problemen: So dürfen keine Veränderungen auf der optischen Uebertragungsstrecke auftreten. Auch Ablagerungen auf der Messsonde oder Fremdobjekte im Reflexionsbereich (Herzwand, Trabekel) führen zu Verfälschungen der Messergebnisse. Ausserdem erfordert dieses Regelprinzip eine Eichung vor der Implantation. Eine erhebliche Verbesserung dieser Situation liesse sich bereits erzielen, wenn ein programmierbarer Herzschrittmacher verwendet würde, der im Dialog auch intrakardiale Messgrößen an eine externe Kontroll- und Steuerstation liefern könnte. Damit könnte unter Umständen in regelmäßigen Abständen oder bei Bedarf eine neue Eichung und damit eine neue Festlegung der Kennlienie vorgenommen werden. Der zu treibende Aufwand wird dafür aber unerwünscht hoch.

Der Erfindung liegt die Aufgabe zugrunde die Schlagfrequenz des Herzens noch besser und schneller den jeweiligen Belastungsverhältnissen anzupassen, wobei die optimale hämodynamische Situation vom Schrittmacher selbst auch über lange Zeiträume unabhänig von Störeinflüssen des Systems, wie teilweise Belegung der Meßsonde oder Verschlechterung der Eigenschaften der optoelektrischen Elemente gefunden wird.

Ein weiteres Ziel der Erfindung ist es, bei der Erfassung der der zentralvenösen Blutsauerstoffsättigung entsprechenden Meßgröße noch besser als bisher Störbeeinflussungen auszuschließen.

Die Lösung dieser Aufgabe erfolgt durch die Merkmale des Anspruchs 1. Weitere Ausgestaltungen der Erfindung sind durch die Merkmale der Unteransprüche gekennzeichnet.

Durch die erfindungsgemäße Regelung ist die Zeitabhängigkeit der Nachlaufregelung beseitigt, weil nicht mehr die Differenzen zweier aufeinanderfolgender Meßwerte, sondern jeweils eines Meßwertes und des maximalen oder minimalen Meßwertes $S_{02max}$ bzw. $S_{02min}$ verwendet werden. Dadurch wird erreicht, daß die gebildete Regelgröße $B_{S1}$ die Information darüber erhält, in welchem Bereich des gesamten Sauerstoffmeßumfanges sich der momentane Meßwert befindet. Anders ausgedrückt bedeutet das, daß in der so gebildeten Regelgröße die Information enthalten ist, welcher Grad der physischen Belastung herrscht. Indem die Stimulationsfrequenz direkt als Funktion der Regelgröße eingestellt wird, erzeugen im Unterschied zu der aus der US-A 4 399 820 bekannten Regelschaltung schnelle Änderungen der Blutsauerstoffsättigung größere Frequenzsprünge als langsame Änderungen, wodurch sich die Herzfrequenz besser als bisher den jeweiligen Belastungsverhältnissen anpaßt. Das entspricht einer Art Kennlinienregelung, jedoch in diesem Fall nicht mehr mit einer fest vorgegebenen Kennlinie wie beim Gegenstand der US-A 4 202 339, sondern mit einer normierten Kennlinie, die der Herzschrittmacher jeweils den vorliegenden Bedingungen im Regelkreis anpaßt. Untersuchungen haben beispielsweise gezeigt, daß sich die maximale Blutsauerstoffsättigungsschwankung im Verlauf längerer Zeiten (Wochen, Monate) stark verändern kann. Im Laufe der Zeit nimmt diese schwankungsbreite ab und außerdem verschiebt sich auch die absolute Lage des Schwankungsbereiches. Die jeweilige Kennlinie kann dabei den gesamten Bereich zwischen minimaler und maximaler Blutsauerstoffsättigung abdecken, aber auch nicht darüber hinausgehen. Durch die erfindungsgemäße Normierung wird das einfach und automatisch sichergestellt.

Im einfachsten Fall wird eine lineare Kennlinie gewählt werden. Wiederum haben jedoch Untersuchungen gezeigt, daß die erforderlichen Frequenzänderungen bei hoher Belastung, d.h. minimaler Blutsauerstoffsättigung, bereits bei geringer Änderung der Blutsauerstoffsättigung recht groß sein müssen, im Ruhezustand dagegen, d.h. bei maximaler Sauerstoffsättigung, kleinere Änderungen der Sauerstoffsättigung kaum einen Einfluß auf die Frequenz haben. Aus diesen Untersuchungen heraus ist in Weiterbildung der Erfindung zur Verbesserung der hämodynamischen Situation vorgesehen, die Regelung nach der Funktion

$$f_p = f_{min} + \Delta f_{max} \exp -c_w((S_{O2}-S_{O2min})/(S_{O2max}-S_{O2min}))^2 + \Delta f_{opt}$$

erfolgt. Die vorgebbare Minimalfrequenz ist dabei mit $f_{min}$, die maximale Frequenzänderung mit $\Delta f_{max}$ bezeichnet. $c_w$ ist eine Konstante und $\Delta f_{opt}$ eine zusätzliche Frequenzänderung durch eine optimierende Regelung. Hierbei ist neben der Kennlinienregelung mit einer normierten, angepassten Kennlinie gleichzeitig und nicht, wie beim Stand der Technik, zeitlich nacheinander, die optimierende Regelung vorgesehen. Kennlinienregelung und optimierende Regelung wirken kontinuierlich nebeneinander, wobei im ersten Fall jedem Blutsauerstoffsättigungswert eine bestimmte Funktion zugeordnet ist und für die optimierende Regelung kontinuierlich die Tendenz des Blutsauerstoffsättigungswertes überwacht und festgestellt wird, ob durch selbstständiges Erhöhen oder Erniedrigen der Frequenz um die Frequenzdifferenz $\Delta f_{opt}$ eine Verbesserung (Messwerterhöhung) des Blutsauerstoffsättigungswertes erfolgt oder nicht.

Jedem Blutsauerstoffsättigungswert ist nicht mehr nur eine bestimmte, sondern eine ganze Schar von Kennlinien zugeordnet. Die obere und untere Grenze ist durch die maximal zugelassene optimierende Regelung festgelegt. Da mit abnehmender maximaler Blutsauerstoffsättigungsschwankungsbreite auch die Messempfindlichkeit (Sensitivität) der Regelschaltung abnimmt, ist in Weiterbildung der Erfindung vorgesehen, dass beim Absinken der Messwertgenauigkeit unter einen vorgebbaren ersten Wert zunächst die optimierende und beim weiteren Absinken unter einen zweiten Wert auch die Kennlinienregelung unterbrochen wird. Damit wird ein einfacher Faktor geschaffen, der es dem Patienten erlaubt festzustellen, wann das Regelsystem in eine kritische Phase gerät und unter Umständen ein Austausch, zumindest aber eine Kontrolle durch den Arzt, erforderlich ist.

Da bei unverändertem Belastungszustand nach einem gewissen Zeitraum durch die optimierende Regelung versuchsweise eine Frequenzänderung vorgenommen wird, reicht es aus, wenn sich der Patient eine Weile in Ruhe befindet und dann durch Pulsmessung feststellt, ob eine Frequenzsprung auftritt oder nicht. Stellt er keine Frequenzerhöhung fest, ist die optimierende Regelung ausser Betrieb.

Um Störungen bei der Messsignalerfassung auszugleichen, ist aus dem Stand der Technik, wie er aus der US-A-4 399 820 bekannt ist, vorgesehen, über einen Integrator den Mittelwert der Messsignale über einen vorgegebenen Zeitraum zu bilden. Wie sich nun überraschend durch Untersuchungen gezeigt hat, können den Messgrössen erhebliche Artefakte überlagert sein, die korreliert zu den Herzschlägen liegen. Die bekannte Mittelwertbildung kann dadurch unter ungünstigen Umständen zu erheblichen Verfälschungen des Messsignals führen. In Weiterbildung der Erfindung ist daher vorgesehen, dass als Messgrösse $S_{O2}$ der Minimalwert des Sensorsignals innerhalb eines oder mehrerer Pulsintervalle verwendet wird. Dieser Minimalwert entspricht der geringsten Blutsauerstoffsättigung und ist damit am besten repräsentativ für den Belastungszustand. Die erwähnten Artefakte können beispielsweise dadurch auftreten, dass durch die Kontraktion des Herzens die Messsonde bewegt wird und beispielsweise in die Nähe der Herzwand gelangt, wodurch die Reflexion und damit das Messsignal erhöht werden.

Erst anschliessend an diese Minimalwertbildung ist es sinnvoll, noch eine Mittelwertbildung vorzunehmen. Da, wie es sich gezeigt hat, die Artefakte nicht stochastisch verteilt liegen, sondern praktisch zeitlich annähernd im Intervall zwischen den Herzschlägen festliegen, und da die Messgrösse nur während eines im Vergleich zum zeitlichen Abstand zweier Herzschläge sehr kurzen Intervalles bestimmt wird, ist zum Erfassen der Artefakte vorgesehen, dass die zeitliche Lage des Messintervalles veränderbar ist. Zumindest im Laufe mehrerer Herzschläge kann damit der gesamte Bereich zwischen diesen abgerastert werden.

Zum schnelleren Erfassen der Artefakte ist es auch möglich, mehrere Zeitintervalle über die Zeitspanne zwischen zwei Herzschlägen zu verteilen. Eine technisch besonders vorteilhafte Lösung ergibt sich dadurch, dass eine grössere Anzahl Messintervalle fest über die Zeitspanne zwischen zwei Herzschlägen verteilt ist, dass aber zum Einsparen von Energie nur jeweils wenige dieser Messintervalle ausgenutzt werden und dass von Herzschlag zu Herzschlag andere der vorhandenen Messintervalle verwendet werden, so dass praktisch eine Phasenverschiebung der verwendeten Messintervalle vorliegt. Wegen der unterschiedlichen Abhängigkeit der Herzschlagfrequenz von der Änderung des Belastungszustandes kann es vorteilhaft sein, die Zahl der pro Herzschlagintervall verwendeten Messintervalle mit steigender Herzschlagfrequenz zu erhöhen.

Anhand von 5 Figuren wird im folgenden ein Ausführungsbeispiel der Erfindung näher beschrieben und erläutert.

Dabei zeigen:

Fig. 1 den zeitlichen Verlauf der maximalen Blutsauerstoffsättigungsschwankung über der Zeit,

Fig. 2 die Änderung der Stimulationsfrequenz abhängig von der Blutsauerstoffsättigung,

Fig. 3 ein Blockschaltbild eines Herzschrittmachers,

Fig. 4 ein Diagramm des zeitlichen Verlaufes der Stimulationsfrequenz in Abhängigkeit von der Blutsauerstoffsättigung und

Fig. 5 ein Diagramm des zeitlichen Verlaufs der Messgrösse sowie der verwendeten Messintervalle in Relation zum EKG.

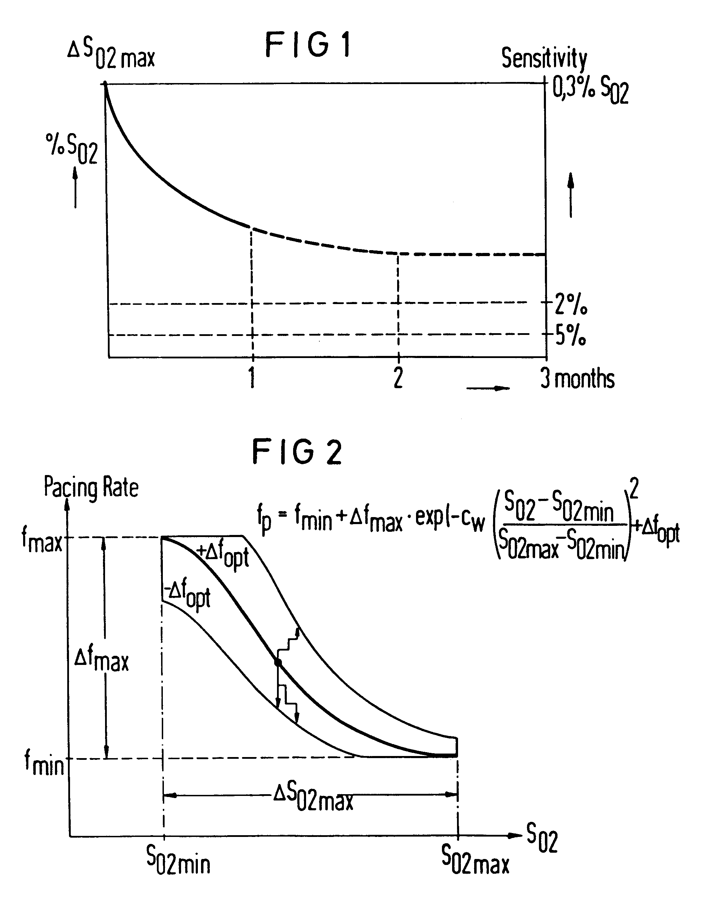

In Fig. 1 ist in einem Diagramm der Verlauf der maximalen Schwankungsbreite der Blutsauerstoffsättigung in Prozent über der Zeit aufgetragen. Zusätzlich ist als weitere Ordinate auf der rechten Seite des Diagrammes die Sensitivität, d.h. die Messgenauigkeit in Prozenten aufgetragen. Diese empirische Kurve zeigt deutlich, dass die maximale Schwankungsbreite der Blutsauerstoffsättigung über längere Zeiträume gesehen abnimmt. Dadurch wird die Messgenauigkeit herabgesetzt, die zu Beginn etwa bei einigen Zehntel Prozent Blutsauerstoffsättigung liegt. Beispielhaft sind hier gestrichelt zwei Grenzwerte für die Sensitivität bei 2 bzw. 5% eingezeichnet. Sinkt die maximale Schwankungsbreite der Blutsauerstoffsättigung $\Delta S_{02max}$ unter die erste Granze von 2% Messgenauigkeit, so kann vorgesehen sein, dass die optimierende Regelung nicht mehr durchgeführt wird. Beim Unterschreiten der unteren Grenze von 5% wird auch die Kennlinienregelung unterbrochen.

Diesem Diagramm ist noch einmal deutlich zu entnehmen, dass eine Kennlinienregelung mit einer fest vorgegebenen, unveränderbaren Kennlinie, die jeweils den gesamten Schwankungsbereich der Blutsauerstoffsättigung frequenzmässig abdecken soll, durch diese starke Veränderung der Schwankungsbreite für die Langzeitregelung nicht geeignet ist.

In dem Diagramm der Fig. 2 ist die Herzschrittmacherfrequenz (pacing rate) über der Blutsauerstoffsättigung $S_{02}$ aufgetragen, wie sie gemäss einem bevorzugten Ausführungsbeispiel der Erfindung vorgenommen werden kann. Daneben ist noch einmal die Funktion der Herzschrittmacherfrequenz $f_p$ in Abhängigkeit von der Blutsauerstoffsättigung angegeben. Wie Versuche gezeigt haben, wird mit dieser Funktion eine optimale Anpassung der Herzfrequenz an die jeweiligen Belastungsverhältnisse erzielt. Im Herz-Schrittmacher ist dazu weiterhin die Minimalfrequenz $f_{min}$ und die maximal zulässige Frequenzänderung $\Delta f_{max}$ festgelegt. Die Funktion setzt sich zusammen aus einem festen Wert für die Minimalfrequenz, einem exponentiellen Glied, das eine einstellbare, insbesondere programmierbare Konstanze $c_w$ und die Regelgrösse $B_{S1}$, sowie ein additives Glied $\Delta f_{opt}$ enthält. Letzteres stellt die Frequenzänderung durch die optimierende Regelung dar. Die erfindungsgemässe Regelung unterscheidet sich von der bekannten Kennlinienregelung in mehrfacher Hinsicht. So wird keine konstante Kennlinie verwendet, sondern eine normierte Kennlinie, die sich den Veränderungen im Regelkreis stets selbstständig anpasst. Weiterhin wird gleichzeitig zur Kennlinienregelung die optimierende Regelung durchgeführt, so dass sich praktisch eine Kennlinienschar ergibt, die hier durch die einhüllenden $+ \Delta f_{opt}$ und $- \Delta f_{opt}$ angedeutet sind. Als Beispiel ist in der Mitte der Kennlinie ein Regelpunkt angegeben. Wenn die gemessene Blutsauerstoffsättigung diesem Punkt entspricht, so wird zunächst die entsprechende Frequenz $f_p$ nach der mittleren Kennlinie eingestellt. Führt die durch die Treppenstufen und Pfeile angedeutete optimierende Regelung zu einer verbesserten Blutsauerstoffsättigung, so wird die bei der optimierenden Regelung vorgenommene Frquenzänderung in bekannter Weise beibehalten. Bei einer anschliessenden Änderung der Belastung erfolgt die Frequenznachregelung daher nach einer

um dieses $\Delta f_{opt}$ verschobenen Kennlinie.

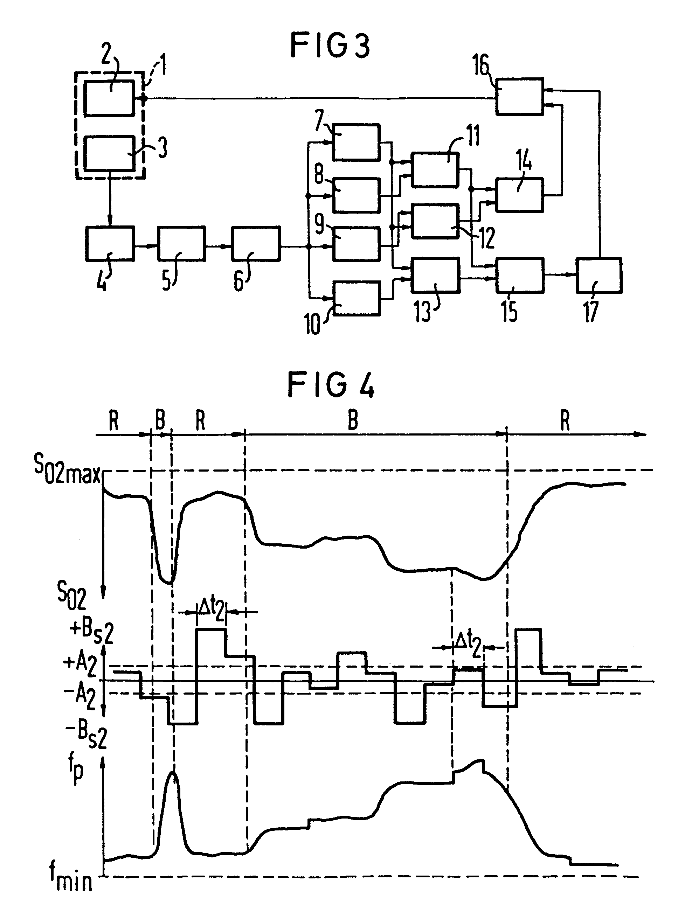

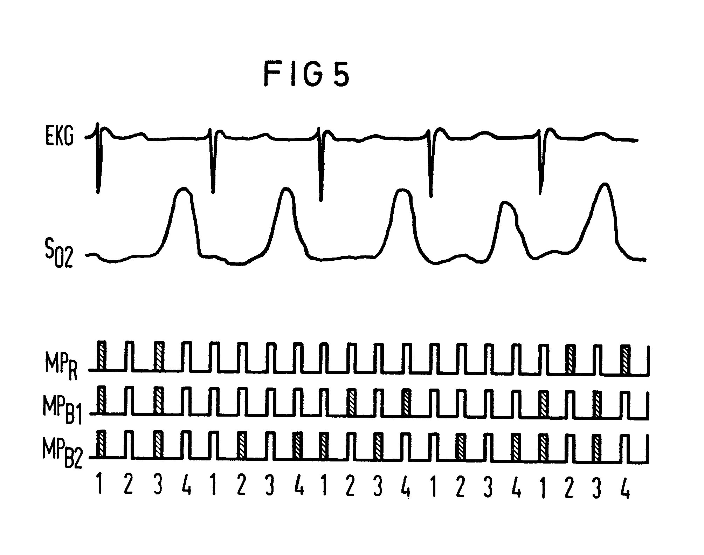

In Fig. 3 sind in einem Blockschaltbild die wichtigsten Funktionen des erfindungsgemässen Schrittmachers dargestellt. Dabei sollen in dem gestrichelt dargestellten Block 1 sowohl die Stimulationselektrode 2 als auch die Messsonde 3 enthalten sein. Das Signal der Messsonde 3 gelangt zunächst auf einen Messverstärker 4 und von dort auf einen Minimalwertbildner 5, der anhand der Fig. 5 später näher erläutert wird. Das Ausgangssignal des Minimalwertbildners gelangt über einen Mittelwertbildner 6 parallel auf vier Speicher 7 bis 10, wobei der Speicher 7 beispielsweise den Maximalwert der Blutsauerstoffsättigung und der Speicher 8 den Minimalwert der Blutsauerstoffsättigung abspeichert.Der Speicher 9 enthält die Signalwerte für die jeweils aktuellen Messzeiten $t_n$ und der Speicher 10 schliesslich den Signalwert zu Messzeiten $t_{n-k}$.

An die Speicher schliessen sich drei Differenzbildner 11 bis 13 an. Im Differenzbildner 11 wird die Messwertdifferenz $\Delta S_{02max}$ zwischen dem über einen längeren Zeitraum erfassten und im Speicher 7 gespeicherten maximalen Blutsauerstoffsättigungswert und im Speicher 8 entsprechend abgespeicherten minimalen Blutsauerstoffsättigungswert gebildet.Im Differenzbildner 12 wird die Differenz des im Speicher 9 vorhandenen neuen Messsignales zum Zeitpunkt $t_n$ und dem Maximalwert der Blutsauerstoffsättigung aus dem Speicher 7 gebildet. Es sei an dieser Stelle betont, dass ebenso die Differenz aus im Speicher 8 vorhandenen minimalen Blutsauerstoffsättigungswert und dem im Speicher 9 vorhandenen neuen Messwert gebildet werden kann.Im Differenzbildner 13 wird entsprechend die Differenz zwischen den Werten der Speicher 7 und 10 gebildet. In den anschliessenden Dividierern 14 bzw. 15 werden aus den Werten der Differenzbildner die normierten Regelgrössen $B_{S1}$ und $B_{S2}$ gebildet.

Der Wert $B_{S1}$ wird direkt auf einen Stimulationsfrequenzgenerator 16 gegeben, der beispielsweise entsprechend den ersten beiden Gliedern der in Fig. 2 angegebenen Funktion in Abhängigkeit von diese Wert eine Herzschrittmacherfrequenz bestimmt. Gleichzeitig wird der Wert $B_{S2}$ auf einen Komparator 17 gegeben, der in bekannter Weise den Wert des Dividierers 15 mit vorgebbaren, insbesondere programmierbaren Festwerten $+A2$ und $-A2$ vergleicht und beim Ueber- bzw. Unterschreiten dieser Werte ebenfalls ein Signal auf den Stimulationsfrequenzgenerator 16 gibt, das zu einem Frequenzsprung um einen konstanten Differenzfrequenzwert führt.

Ueber das Ausgangssignal des Stimulationsfrequenzgenerators wird die Stimulationselektrode angesteuert.

In der folgenden Fig. 4 ist die Regelung der Herzschrittmacherstimulationsfrequenz $f_p$ mit Hilfe der Regelschaltung gemäß einem Ausführungsbeispiel der Erfindung in Abhängigkeit von der Belastung des Patienten dargestellt durch den Zusammenhang im zeitlichen Verlauf des Messwertes der Blutsauerstoffsättigung $S_{02}$, dessen Veränderung pro Zeiteinheit $\Delta t_2$ sowie der dadurch bewirkten Änderung der Frequenz $f_p$. Im oberen Teil der Fig. 4 sind dabei mit den Buchstaben R bzw. B Phasen der Ruhe bzw. Phasen der Belastung gekennzeichnet. Darunter ist die Blutsauerstoffsättigung aufgetragen. Im mittleren Bereich der Fig. 4 ist der Verlauf der Regelgrösse $B_{S2}$ für die optimierende Regelung dargestellt sowie gestrichelt die beiden Festwerte $+A_2$ und $-A_2$. Im unteren Teil der Fig. 4 ist der Verlauf der Herzschlagfreqenz $f_p$ aufgezeichnet. Wie man dieser Darstellung deutlich entnehmen kann, sinkt bei Beginn der ersten Belastungsphase die zentralvenöse Sauerstoffsättigung $S_{02}$ rasch ab. Fast ohne Verzögerung erfolgt dadurch eine Erhöhung der Herzfrequenz $f_p$. Das wird durch die vorgesehene Nachregelung gemäß einem Ausführungsbeispiel der Erfindung über die normierte Kennlinie mit überlagerter optimierender Regelung erreicht. In der daran anschliessenden Ruhephase geht die Sauerstoffsättigung wieder auf ihren anfänglichen höheren Wert zurück. Das ist wiederum mit einem nahezu gleichzeitigen, entsprechenden Absinken der Herzschrittmacherfrequenz verbunden.

In der zweiten dargestellten und sehr viel längeren Belastungsphase B tritt, wenn der Sauerstoffsättigungswert annähernd einen konstanten Wert annimmt, durch die optimierende Regelung eine kleine Frequenzerhöhung der Herzschrittmacherfrequenz auf. Diese führt, wie man dem Kurvenverlauf über die Blutsauerstoffsättigung entnehmen kann, kurz nach der Frequenzänderung zu einer verbesserten Blutsauerstoffsättigung. Diese Frequenzänderung wird daher beibehalten. Im Endbereich dieser Belastungsphase wird noch einmal eine optimierende Frequenzänderung vorgenommen, die sich wieder in dem Frequenzsprung in der unteren Kurve ausweist. Diesmal erfolgt jedoch keine Verbesserung der Blutsauerstoffsättigung. Der unmittelbar anschliessenden steigenden Belastung - der absinkenden Blutsauerstoffsättigung - folgt die Frequenz wieder direkt. Am Ende dieses Zeitintervalles $\Delta t_2$ wird die Frequenzänderung durch die optimierende Regelung wieder zurückgenommen.

In der abschliessenden Fig. 5 ist im oberen Teil ein EKG dargestellt. Darunter ist der Verlauf der Messgrösse der Blutsauerstoffsättigung $S_{02}$ dargestellt. Im unteren Teil dieser Fig. 5 sind die Messpunkte dargestellt,bei denen jeweils im Verlauf des EKG's die Blutsauerstoffsättigung bestimmt wird. Mit $MP_R$ sind dabei die schwarz eingezeichneten Messpunkte im Ruhezustand des Patienten dargestellt, mit $MP_{B1}$ in einem ersten, mittleren Belastungszustand und mit $MP_{B2}$ in einem zweiten,höheren Belastungszustand.Dabei ist angenommen, dass in jedem Herzzykluz vier Messpunkte möglich sind, dass aber zur Einsparung von Energie nicht sämtliche Messpunkte ausgenutzt werden.Wie man im mittleren Teil dieser Figur bei der Darstellung der Blutsauerstoffsättigung deutlich erkennen kann, ist diese Messkurve durch Artefakte gestört, die mit grosser Amplitude und korreliert zu den Herzaktivitäten auftreten. Eine reine Mittelwertbildung des Messwertes würde daher in vielen Fällen zu falschen Messergebnissen und damit zu einer falschen Regelung führen.

In Weiterbildung der Erfindung ist daher vorgesehen, dass zunächst einmal die Messpunkte von Herzzyklus zu Herzzyklus zeitlich anders liegen, so dass auch bei der Verwendung weniger Messpunkte in einem Zyklus über mehrere Herzzyklen gesehen der gesamte Zyklus abgetastet und die Artefakte erfasst werden. Dabei wird bei der Ruhe oder geringen Belastung nicht einmal in jedem Herzzyklus gemessen. Erst bei sehr hoher Belastung wird wegen der starken Abhängigkeit der Frequenz von Änderung der Blutsauerstoffsättigung mehrfach in jedem Zyklus gemessen. Aus den so erhaltenen Messwerten wird dann zunächst der Minimalwert gebildet.Damit fallen die starken Störungen durch die Artefakte heraus. Wie sich gezeigt hat, führen bei den optoelektrischen Messonden Artefakte stets zu einer Erhöhung des Blutsauerstoffwertes. Der Minimalwert ist daher eine sinnvolle Messgrösse für den tatsächlichen Belastungszustand. Nach der Minimalwertbildung kann, wie aus da Stand der Technik bekannt, noch eine Mittelwertbildung angeschlossen werden.

Auch bei dieser Regelschaltung kann der Herzschrittmacher inhibiert arbeiten, d.h. bei Herzeigenerregung die Stimulation durch den Stimulationsfrequenzgenerator verhindern.

**Patentansprüche**

1. Regelschaltung zur Anpassung der Stimulationsfrequenz $f_p$ eines Herzschrittmachers an die Belastung eines Patienten, bei der eine der zentralvenösen Blutsauerstoffsättigung im Herzen entsprechende Meßgröße $S_{02}$ nach dem Prinzip der Reflexionsoximetrie in aufeinanderfolgenden Zeitintervallen ermittelt wird, bei der die ermittelte Meßgröße $S_{02}$ zur Bildung einer Regelgröße $B_{s1}$ mit der maximalen Änderung $\Delta S_{02max} = S_{02max} - S_{02min}$ der Meßgröße $S_{02}$ innerhalb aufeinanderfolgender und in bezug auf die Zeitintervalle langer Zeitabstände normiert wird und bei der die Regelgröße $B_{S1}$ zur Nachlaufregelung der Stimulationsfrequenz $f_p$ herangezogen wird, **dadurch gekennzeich-**

**net,** daß die Regelgröße $B_{S1}$ in Form des Quotienten $B_{S1} = \Delta S_{02}/\Delta S_{02max}$ gebildet wird, wobei $\Delta S_{02}$ die Differenz zwischen der ermittelten Meßgröße $S_{02}$ und dem Minimalwert $S_{02min}$ oder Maximalwert $S_{02max}$ ist, und daS die Stimulationsfrequenz $f_p$ direkt als Funktion der Regelgröße $B_{S1}$ eingestellt wird.

2. Regelschaltung nach Anspruch 1, **dadurch gekennzeichnet**, daß ferner Änderungen $\Delta S_{02opt}$ der Meßgröße $S_{02}$ in zweiten, aufeinanderfolgenden Zeitintervallen $\Delta t_2$ bestimmt werden und eine weitere Regelgröße $B_{S2}$ in Form des Quotienten $B_{S2} = \Delta S_{02opt}/\Delta S_{02max}$ gebildet wird und daß in einer gleichzeitig mit der Kennlinienregelung vorgenommenen optimierenden Regelung die Stimulationsfrequenz $f_p$ zunächst um eine Frequenzdifferenz $\Delta f_{opt}$ erhöht oder erniedrigt wird, wobei anschließend die Änderung der Stimulationsfrequenz $f_p$ nur dann aufrecht erhalten wird, wenn durch die Frequenzänderung eine vorgegebene Erhöhung der Meßgröße $S_{02}$ erreicht oder überschritten wird.

3. Regelschaltung nach Anspruch 2, **dadurch gekennzeichnet**, daß die Regelung der Stimulationsfrequenz $f_p$ nach der Funktion $f_p = f_{min} + \Delta f_{max} * \exp(-c_w B_{S1}^2) + \Delta f_{opt}$ erfolgt, wobei $f_{min}$ eine vorgegebene Minimalfrequenz, $\Delta f_{max}$ die maximale Frequenzänderung und $c_w$ eine Konstante ist.

4. Regelschaltung nach Anspruch 3, **dadurch gekennzeichnet**, daß die Parameter $f_{min}$, $\Delta f_{max}$, $c_w$ und $\Delta f_{opt}$ einstellbar und insbesondere programmierbar sind.

5. Regelschaltung nach einem der Ansprüche 2 bis 4, **dadurch gekennzeichnet**, daß beim Absinken der Meßwertgenauigkeit (Sensitivität) unter einen vorgebbaren ersten Wert die optimierende und beim weiteren Absinken unter einen zweiten Wert die Nachlaufregelung abschaltbar sind.

6. Regelschaltung nach einem der vorangehenden Ansprüche, **dadurch gekennzeichnet**, daß als Meßgröße $S_{02}$ der Mimimalwert mehrerer Meßwerte innerhalb eines bestimmten Zeitintervalls, vorzugsweise einer Pulsdauer, verwendet wird.

7. Regelschaltung nach einem der vorangehenden Ansprüche, **dadurch gekennzeichnet**, daß die Meßwerte nur während eines im Vergleich zum zeitlichen Abstand zweier Herzschläge (QRS-Komplex) sehr kurzen Meßintervalles bestimmt werden, das seine zeitliche Lage zu den Herzschlägen ändert.

8. Regelschaltung nach Anspruch 7, **dadurch gekennzeichnet**, daß mehrere mögliche Meßintervalle über die Zeitspanne zwischen den Herzschlägen verteilt sind.

9. Regelschaltung nach Anspruch 7, **dadurch gekennzeichnet**, daß die Meßintervalle mit den Herzschlägen getriggert sind und von den möglichen Intervallen nach jedem Herzschlag die ausgenutzten zu denen der vorangegangenen Zeitspanne phasenverschoben sind.

## Claims

1. Control circuit for matching the stimulation frequency $f_p$ of a heart pacemaker to the load condition of a patient, in which a measured variable $S_{02}$, corresponding to the central venous blood oxygen saturation in the heart, is determined according to the principle of reflection oximetry in successive time intervals, in which the measured variable $S_{02}$ determined for creating a control variable $B_{S1}$ the maximum change $\Delta S_{02max} = S_{02max} - S_{02min}$ of the measured variable $S_{02}$ is normalised within time periods with which are successive and are long in comparison with the time intervals, and in which the control parameter $B_{S1}$ is made use of for follow-up control of the stimulation frequency $f_p$, characterised in that the control variable $B_{S1}$ is formed as the quotient $B_{S1} = \Delta S_{02}/\Delta S_{02max}$, $\Delta S_{02}$ being the difference between the measured variable $S_{02}$ determined and the minimum value $S_{02min}$ or maximum value $S_{02max}$, and in that the stimulation frequency $f_p$ is set directly as a function of the control variable $B_{S1}$.

2. Control circuit according to Claim 1, characterised in that, furthermore, changes $\Delta S_{02opt}$ in the measured variable $S_{02}$ are defined in second, successive time intervals $\Delta t_2$ and a further control variable $B_{S2}$ is formed as the quotient $B_{S2} = \Delta S_{02opt}/\Delta S_{02max}$ and in that the stimulation frequency $f_p$ is initially increased or decreased by a frequency difference $\Delta f_{opt}$, in an optimising control carried out at the same time as the control of the characteristic, the change in the stimulation frequency $f_p$ subsequently being maintained only if a predetermined increase in the measured variable $S_{02}$ is achieved or exceeded as a result of the frequency change.

3. Control circuit according to Claim 2, characterised in that the stimulation frequency $f_p$ is controlled in accordance with the function $f_p = f_{min} + \Delta f_{max} \times \exp(-c_w B_{S1}^2) + \Delta f_{opt}$, where $f_{min}$ is a predetermined minimum frequency, $\Delta f_{max}$ is the maximum frequency change and $c_w$ is a constant.

4. Control circuit according to Claim 3, characterised in that the parameters $f_{min}$, $\Delta f_{max}$, $c_w$ and $\Delta f_{opt}$ are adjustable and preferably programmable.

5. Control circuit according to one of Claims 2 to 4, characterised in that the optimising control can be switched off in the event of the measured value accuracy (sensitivity) falling below a predetermined first value and the follow-up control can be switched off in the event of said accuracy falling further, below a second value.

6. Control circuit according to one of the preceding claims, characterised in that the minimum value of a plurality of measured values within a specific time interval, preferably the duration of one pulse, is used as the measured variable $S_{02}$.

7. Control circuit according to one of the preceding claims, characterised in that the measured values are defined only during a measurement interval which is very short in comparison with the time period between two heart beats (QRS complex) and which changes its position in time with respect to the heart beats.

8. Control circuit according to Claim 7, characterised in that a plurality of possible measurement intervals are distributed over the time period between the heart beats.

9. Control circuit according to Claim 7, characterised in that the measurement intervals are triggered using the heart beats and, of the possible intervals after each heart beat, those used are phase-shifted with respect to those of the previous time period.

## Revendications

1. Circuit de régulation pour adapter la fréquence de stimulation $f_p$ d'un stimulateur cardiaque à la charge d'un patient et dans lequel une grandeur de mesure $S_{02}$, qui correspond à la saturation en oxygène dans le sang du système veineux central dans le coeur, est déterminée selon le principe de l'oxymétrie à réflexion à des intervalles de temps successifs, et dans lequel la grandeur de mesure déterminée $S_{02}$

est normalisée pour former une grandeur de régulation $B_{S1}$, par la variation maximale $\Delta_{S02max} = S_{02max} - S_{02min}$ de la grandeur de mesure $S_{02}$ pendant des durées successives supérieures aux intervalles de temps, et dans lequel la grandeur de régulation $B_{S1}$ est utilisée pour régler par asservissement la fréquence de stimulation $f_p$, caractérisé par le fait que la grandeur de régulation $B_{S1}$ est formée en tant que quotient $B_{S1} = \Delta S_{02}/\Delta S_{02max}$, $\Delta S_{02}$ représentant la différence entre la grandeur de mesure déterminée $S_{02}$ et la valeur minimale $S_{02min}$ ou la valeur maximale $S_{02max}$, et que la fréquence de stimulation $f_p$ est réglée directement en fonction de la grandeur de régulation $B_{S1}$.

2. Circuit de régulation suivant la revendication 1, caractérisé par le fait qu'en outre des variations $\Delta S_{02opt}$ de la grandeur de mesure $S_{02}$ sont déterminées pendant des seconds intervalles de temps successifs $\Delta t_2$ et qu'une autre grandeur de régulation $B_{S2}$ est formée en tant que quotient $B_{S2} = \Delta S_{02opt}/\Delta S_{02max}$, et que lors d'une régulation d'optimisation exécutée en même temps que la régulation de la courbe caractéristique, la fréquence de stimulation $f_p$ est tout d'abord accrue ou réduite d'une différence de fréquence $\Delta f_{opt}$, la variation de la fréquence de stimulation $f_p$ étant ensuite maintenue uniquement lorsque, par la variation de la fréquence, un accroissement prédéterminé de la grandeur de mesure $S_{02}$ est atteint ou est dépassé.

3. Circuit de régulation suivant la revendication 2, caractérisé par le fait que la régulation de la fréquence de stimulation $f_p$ est réalisée conformément à la fonction $f_p = f_{min} + \Delta f_{max} * \exp(-c_w B_{S1}^2) + \Delta f_{opt}$, $f_{min}$ étant une fréquence minimale prédéterminée, $\Delta f_{max}$ la variation maximale de fréquence et $c_w$ une constante.

4. Circuit de régulation suivant la revendication 3, caractérisé par le fait que les paramètres $f_{min}$, $\Delta f_{max}$, $c_w$ et $\Delta f_{opt}$ sont réglables et, notamment, programmables.

5. Circuit de régulation suivant l'une des revendications 2 à 4, caractérisé par le fait que lorsque la précision de la valeur de mesure (sensibilité) tombe au-dessous d'une première valeur pouvant être prédéterminée, la régulation d'optimisation peut être interrompue et lorsque la pression tombe au-dessous d'une seconde valeur, la régulation par asservissement peut être interrompue.

6. Circuit de régulation suivant l'une des revendications précédentes, caractérisé par le fait qu'on utilise comme grandeur de mesure $S_{02}$ la valeur minimale de plusieurs valeurs pendant un intervalle de temps déterminé, de préférence une durée d'impulsion.

7. Circuit de régulation suivant l'une des revendications précédentes, caractérisé par le fait que les valeurs de mesure sont déterminées uniquement pendant un intervalle de mesure qui est très bref par rapport à l'intervalle de temps entre le battement cardiaque (complexe QRS), et dont la position dans le temps par rapport au battement cardiaque varie.

8. Circuit de régulation suivant la revendication 7, caractérisé par le fait que plusieurs intervalles de mesure possibles sont répartis dans l'intervalle de temps s'étendant entre les battements cardiaques.

9. Circuit de régulation suivant la revendication 7, caractérisé par le fait que les intervalles de mesure sont déclenchés par les battements cardiaques et que ceux des intervalles de temps possibles, qui sont utilisés, après chaque battement cardiaque, sont déphasés par rapport aux intervalles de temps apparus pendant la durée précédente.

# FIG 1

$\Delta S_{O2\,max}$

$\%\,S_{O2}$

Sensitivity

0,3% $S_{O2}$

2%

5%

1      2      3 months

# FIG 2

Pacing Rate

$$f_p = f_{min} + \Delta f_{max} \cdot exp\left[-c_w \left(\frac{S_{O2} - S_{O2\,min}}{S_{O2\,max} - S_{O2\,min}}\right)^2\right] + \Delta f_{opt}$$

$f_{max}$

$+\Delta f_{opt}$

$-\Delta f_{opt}$

$\Delta f_{max}$

$f_{min}$

$\Delta S_{O2\,max}$

$S_{O2}$

$S_{O2\,min}$        $S_{O2\,max}$

# FIG 3

# FIG 4

FIG 5